# EUROPÄISCHE PATENTSCHRIFT

(11) **EP 1 627 617 B1**
(45) Veröffentlichungstag und Bekanntmachung des Hinweises auf die Patenterteilung: **27.08.2008**
(21) Anmeldenummer: 05025609.8
(22) Anmeldetag: 10.06.2003
(51) Int. Cl.: A61F 9/009, A61F 9/01

(54) **Ophthalmologische Vorrichtung für die Auflösung von Augengewebe**
Ophthalmological device for ablation of eye tissue
Dispositif ophthalmologique destiné à l'ablation des tissus des yeux

(43) Veröffentlichungstag der Anmeldung: 22.02.2006
(62) Teilanmeldung aus: 03405415.5
(73) Patentinhaber: SIE AG, Surgical Instrument Engineering, 2562 Port (CH)
(72) Erfinder: Rathjen, Christian, 28197 Bremen (DE)
(74) Vertreter: Vogel, Dany

(56) Entgegenhaltungen:
- WO-A-89/06519
- WO-A-99/65431
- DE-A- 19 831 674
- US-A- 4 665 913
- US-A- 5 549 632
- US-B1- 6 494 878

## Beschreibung

### Technisches Gebiet

Die vorliegende Erfindung betrifft eine ophthalmologische Vorrichtung für die Auflösung von Augengewebe. Die Erfindung betrifft insbesondere eine ophthalmologische Vorrichtung, welche einen Handgriff zum manuellen Halten und Applizieren der ophthalmologischen Vorrichtung umfasst, welche Befestigungsmittel zum Fixieren der ophthalmologischen Vorrichtung an einem Auge umfasst, und welche eine Lichtquelle und einen mit der Lichtquelle optisch verbundenen Lichtprojektor zur fokussierten Projektion von Lichtpulsen für die punktuelle Gewebeauflösung an einem Brennpunkt im Innern des Augengewebes umfasst.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem computergesteuerten Eximerlaser abgetragen wird, nachdem zuvor ein Hornhautlappen teilweise abgetrennt und weggeklappt wird. Zur Erzeugung des Hautlappens werden mechanische Mikrokeratome eingesetzt, bei denen ein angetriebenes Messer den Hautlappen schneidet. Neuerdings können solche Hautlappen auch mit stark fokussierten Femtosekundenlaserpulsen geschnitten werden, die Pulsbreiten von typisch 100fs bis 1000fs (1fs=10⁻¹⁵S) aufweisen. Die beim Einsatz eines mechanisch oszillierenden Messers bestehenden Gefahren werden bei der Verwendung eines Femtosekundenlasers vermieden. Ein solches System wird von der Firma IntraLase Corp, in Irvine, California, USA, unter dem Namen Pulsion FS Laser angeboten. Die Baugrösse der bekannten Systeme mit Femtosekundenlaser ist mit der Baugrösse eines Eximerlasers vergleichbar, was den Nachteil hat, dass im Behandlungsraum der Platz, der für den Eximerlaser benötigt wird, ein weiteres Mal für das Femtosekundenlasersystem benötigt wird. Zudem muss der Patient nach dem Schneiden des Hautlappens vom Femtosekundenlasersystem zum Eximerlaser transferiert werden. Die Baugrösse der Femtosekundenlaser wird unter anderem durch die verwendete Lichtquelle, die Scannertechnologie und die damit einhergehenden Strahlführungssysteme bestimmt. Innerhalb eines grossen stationären Linsensystems wird der Laserstrahl durch strahlumlenkende optische Elemente auf die zu trennenden Gewebebereiche des Auges fokussiert. Konstruktionsbedingt ist dabei die maximal erreichbare numerische Apertur (NA proportional zum halben Sinus des Öffnungswinkel des Objektivs) der Fokussieroptik begrenzt (typisch NA=0.2-0.3). Bei gegebenem Arbeitsfeld, beispielsweise die gesamte Hornhaut, erfordert die Scanoptik (typischerweise eine f-Theta Optik) einen Mindestarbeitsabstand. Aus dem Arbeitsabstand ergibt sich im Zusammenhang mit der erforderlichen Bewegung und der erreichbaren Grösse der strahlumlenkenden optischen Elemente eine konstruktive Grenze für den Durchmesser der Scanoptik. Eine weitere konstruktive Obergrenze für den Durchmesser ergibt sich durch Abschattungen beziehungsweise Kollisionen mit Körperteilen (Augenbrauen, Nase). Auch bei grossen Durchmessern kann immer nur ein Teilbereich der Optik vom scannenden Laserstrahl ausgeleuchtet werden. Damit ergibt sich eine konstruktive Obergrenze für die effektive ausnutzbare numerische Apertur der Optik. Hohe Aperturen sind wünschenswert, weil sich mit hohen NA kleine Brennpunkte und damit eine kleinere Schnittzone pro Puls erzeugen lassen. In kleineren Schnittzonen wird pro Puls weniger Gas erzeugt, als in grossen Schnittzonen. Durch kleinere Gasblasen lassen sich präzisere Schnitte erzeugen, da unter anderem die Schnittzonen nicht durch den internen Gasdruck deformiert werden. Zudem benötigen hohe NA überproportional weniger Energie pro Puls zur Erzeugung eines Schnittes. Mit geringerer Energie reduzieren sich auch die durch den Laserpuls erzeugten Kavationsblasen, was sich zusätzlich positiv auf die Schnittqualität auswirkt. Weiterhin wird die Netzhaut durch die stärker divergierenden Strahlen hinter dem Brennpunkt bei hoher NA weniger belastet. Ein weiterer Vorteil ist, dass bei hoher NA lokale Verschmutzungen in der Nähe der Hornhautoberfläche die Intensität im Fokus weniger reduzieren.

In der Patentschrift US 5,549,632 wird eine ophthalmologische Vorrichtung mit einer Laserquelle für die Auflösung von Augengewebe beschrieben, welche unter anderem für das Schneiden von Hornhautlappen einsetzbar ist. Die Vorrichtung gemäss US 5,549,632 umfasst eine Laserquelle sowie einen mit der Laserquelle optisch verbundenen Projektionskopf in einem von der Laserquelle separaten Gehäuse. Die Vorrichtung gemäss US 5,549,632 umfasst zudem Strahlsteuermittel, die den Strahlengang der von der Laserquelle abgegebenen Laserpulse so steuern, dass Punkte in einem zur Laserquelle festen Referenzrahmen über eine optische Verbindung auf entsprechende Punkte in einem zum Projektionskopf festen Referenzrahmen abgebildet werden. Damit die durch die Strahlsteuermittel abgelenkten Lichtpulse relativ zum Referenzrahmen des Handgeräts abgebildet werden können, ist die optische Verbindung aufwendig als Spiegelgelenkarm ausgeführt. Durch die Verbindung des Projektionskopfs mit einer fest mit dem Auge verbindbaren Applanationsplatte wird der feste Referenzrahmen des Projektionskopfs gemäss US 5,549,632 fest auf die Applanationsplatte und somit auf das Auge abgebildet. Gemäss US 5,549,632 werden die Laserpulse an gewünschte Positionen des Auges geleitet, indem die Strahlsteuermittel die Position des gepulsten Laserstrahls relativ zu der Applanationsplatte steuern und über die optische Verbindung und das optische Projektionssystem des Projektionskopfs auf das Auge abbilden. Um beispielsweise Schnitte von 5 bis 15 mm Länge durchzuführen muss das optische Projektionssystem des Projektionskopfs optische Linsen aufweisen, deren Durchmesser grösser als der Durchmesser des Augapfels ist. Durch einen derart gross dimensionierten Projektionskopf wird die Sicht auf das zu behandelnde Auge verdeckt. Weiterhin ist die numerische Apertur der Vorrichtung gemäss US 5,549,632 gering, wie aus der geringeren Konvergenz der Strahlen ersichtlich ist.

In der Patentschrift US 4,901,718 wird eine weitere ophthalmologische Vorrichtung zur Fokussierung von Laserpulsen auf das Auge beschrieben. Die Position der Laseroptik kann gemäss US 4,901,718 relativ zum Auge verändert werden. Auch die Vorrichtung gemäss US 4,901,718 erreicht nur eine geringe numerische Apertur, was durch die Wahl der Scanmethode und der Strahlführung bedingt ist. Wie in der Vorrichtung gemäss US 5,549,632 ist der gerätetechnische Aufwand zur Strahlführung auch in der Vorrichtung gemäss US 4,901,718 erheblich.

Grosse Linsensysteme haben zudem den Nachteil, dass durch sie Vorrichtungen schwer und unhandlich werden, wodurch manuelles Halten und Applizieren erschwert wird.

### Darstellung der Erfindung

Es ist eine Aufgabe der vorliegenden Erfindung, eine neue ophthalmologische Vorrichtung für die Auflösung von Augengewebe vorzuschlagen, welche mindestens gewisse Nachteile des Stands der Technik nicht aufweist und welche insbesondere für die punktuelle Gewebeauflösung im Innern des Augengewebes geeignet ist.

Gemäss der vorliegenden Erfindung werden diese Ziele insbesondere durch die Elemente des unabhängigen Anspruchs erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Vorrichtung, die einen Handgriff zum manuellen Halten und Applizieren der ophthalmologischen Vorrichtung, Befestigungsmittel zum Fixieren der ophthalmologischen Vorrichtung an einem Auge, beispielsweise durch Unterdruck, und eine Lichtquelle und einen mit der Lichtquelle optisch verbundenen Lichtprojektor zur fokussierten Projektion von Lichtpulsen für die punktuelle Gewebeauflösung an einem Brennpunkt im Innern des Augengewebes umfasst, mit einem Bewegungstreiber zur Bewegung des Lichtprojektors versehen ist. Der Bewegungstreiber ermöglicht durch die mechanische Bewegung des Lichtprojektors in vorteilhafter Weise den Brennpunkt für die punktuelle Gewebeauflösung automatisiert an mehrere Stellen im Innern des Augengewebes zu bewegen. Weil der Brennpunkt unter Zuhilfenahme des Bewegungstreibers bewegt wird, kann das optische Projektionssystem des Lichtprojektors, bei gleichem Aktionsbereich der ophthalmologischen Vorrichtung, wesentlich kleiner dimensioniert werden als bei einer ophthalmologischen Vorrichtung, bei der der Brennpunkt ausschliesslich durch das optische Projektionssystem bewegt wird. Durch die derartige Miniaturisierung des optischen Projektionssystems kann zudem eine schärfere Fokussierung der Lichtpulse, das heisst eine schärfere Abbildung der Lichtpulse am Brennpunkt im Innern des Augengewebes, erreicht werden, da sich bei der Applikation der ophthalmologischen Vorrichtung auf das Auge die Distanz zwischen dem optischen Projektionssystem und dem Auge verkleinert und sich ein kleinerer Arbeitsabstand als bei Systemen mit ausschliesslich optisch bewegtem Brennpunkt ergibt und sich dadurch bei kleineren Dimensionen eine höhere NA ergibt. Die schärfere Fokussierung der Lichtpulse ermöglicht präzisere Behandlungen mit glätteren Schnittflächen und eine geringere Belastung für das Auge.

Vorzugsweise sind der Bewegungstreiber und der Lichtprojektor so eingerichtet und gekoppelt, dass der Lichtprojektor in eine Position bewegbar ist, in welcher beim Fixieren der ophthalmologischen Vorrichtung am Auge mindestens ein Teil des Auges in einer Aufsicht einsehbar ist. Dadurch ist es für den Benutzer möglich, die ophthalmologische Vorrichtung bei freier Sicht auf das zu behandelnde Auge an diesem Auge zu fixieren.

Vorzugsweise sind der Bewegungstreiber und der Lichtprojektor so eingerichtet und gekoppelt, dass der Lichtprojektor äquidistant zu einer Bewegungsfläche bewegbar ist. Dadurch kann der Brennpunkt für die punktuelle Gewebeauflösung durch den Bewegungstreiber so bewegt werden, dass im Innern des Augengewebes Schnitte und Schnittflächen äquidistant zu der Bewegungsfläche erzeugt werden.

Vorzugsweise sind der Bewegungstreiber und der Lichtprojektor so eingerichtet und gekoppelt, dass der Lichtprojektor äquidistant zu einer ebenen Bewegungsfläche bewegbar ist. Dadurch kann der Brennpunkt für die punktuelle Gewebeauflösung durch den Bewegungstreiber so bewegt werden, dass im Innern des Augengewebes Schnitte und ebene Schnittflächen äquidistant zu einer Bewegungsebene erzeugt werden.

In einer Ausführungsvariante ist der Bewegungstreiber eingerichtet, den Lichtprojektor entlang einer zur Bewegungsfläche äquidistanten Translationslinie zu bewegen. Insbesondere im Fall einer ebenen Bewegungsfläche können so durch einfache mechanische Verschiebung des Lichtprojektors entlang einer äquidistanten Translationsachse im Innern des Augengewebes gerade Schnitte erzeugt werden. In einer Ausführungsvariante umfasst die ophthalmologische Vorrichtung einen zweiten solchen Bewegungstreiber, der eingerichtet ist, den Lichtprojektor entlang einer zur Bewegungsfläche äquidistanten zweiten Translationslinie zu bewegen. Durch die Kombination zweier translatorischer Bewegungstreiber können so durch einfache mechanische Verschiebungen des Lichtprojektors entlang zweier Translationslinien im Innern des Augengewebes Schnittflächen äquidistant zu der Bewegungsfläche erzeugt werden, im Falle der ebenen Bewegungsfläche können so ebene Schnittflächen erzeugt werden.

In einer Ausführungsvariante ist der Bewegungstreiber eingerichtet, den Lichtprojektor um eine zur Bewegungsfläche normale Rotationsachse zu bewegen. Dadurch kann der Brennpunkt für die punktuelle Gewebeauflösung durch den Bewegungstreiber so bewegt werden, dass im Innern des Augengewebes kreisförmige Schnitte äquidistant zu der (ebenen) Bewegungsfläche erzeugt werden.

In einer Ausführungsvariante ist der Bewegungstreiber eingerichtet, den Lichtprojektor entlang einer zur Bewegungsfläche äquidistanten Translationslinie zu bewegen und die ophthalmologische Vorrichtung umfasst einen weiteren Bewegungstreiber, der eingerichtet ist den Lichtprojektor um eine zur Bewegungsfläche normale Rotationsachse zu bewegen. Insbesondere im Fall einer ebenen Bewegungsfläche können so durch die Kombination von Verschiebung und Rotation des Lichtprojektors im Innern des Augengewebes ebene Schnittflächen ähnlich wie durch Fräsen erzeugt werden.

Vorzugsweise ist die ophthalmologische Vorrichtung eingerichtet, den Abstand zwischen dem Brennpunkt und der Bewegungsfläche zu regeln. Durch die Veränderung des Abstands zwischen dem Brennpunkt und der Bewegungsfläche kann der Brennpunkt für die punktuelle Gewebeauflösung so bewegt werden, dass im Innern des Augengewebes Schnitte und Schnittflächen erzeugt werden, die nicht äquidistant zu der Bewegungsfläche sind und die beispielsweise senkrecht zur der Bewegungsfläche sind.

In einer Ausführungsvariante ist die ophthalmologische Vorrichtung eingerichtet, die Position des Brennpunkts entlang der Projektionsachse der Lichtpulse zu regeln. Durch Veränderung der Position des Brennpunkts entlang der Projektionsachse der Lichtpulse kann der Abstand zwischen dem Brennpunkt und der Bewegungsfläche verändert werden, wodurch, wie oben stehend beschrieben, im Innern des Augengewebes Schnitte und Schnittflächen erzeugt werden, die nicht äquidistant zu der Bewegungsfläche sind und die beispielsweise senkrecht zur der Bewegungsfläche sind.

In einer Ausführungsvariante umfasst der Lichtprojektor lichtumlenkende optische Elemente zur fokussierten Projektion der Lichtpulse entlang einer Scanlinie. Durch die Verwendung von lichtumlenkenden optischen Elementen, typischerweise bewegliche lichtumlenkende optische Elemente, kann der Brennpunkt optisch so bewegt werden, dass im Innern des Augengewebes Gewebe punktuell entlang der Scanlinie aufgelöst wird. Durch die Kombination dieser optischen Bewegung des Brennpunkts mit den oben beschriebenen translatorischen oder rotatorischen mechanischen Bewegungen des Lichtprojektors können wiederum im Innern des Augengewebes Schnittflächen äquidistant zu der Bewegungsfläche, respektive zu der Bewegungsebene, erzeugt werden.

Vorzugsweise umfasst die ophthalmologische Vorrichtung einen auf das Auge aufsetzbaren, mindestens stellenweise lichtdurchlässigen Kontaktkörper, welcher so ausgebildet und angeordnet ist, dass er einen im aufgesetzten Zustand kontaktierten Bereich des Auges äquidistant zu der Bewegungsfläche setzt. Da der Brennpunkt für die punktuelle Gewebeauflösung im Innern des Augengewebes äquidistant zu der Bewegungsfläche bewegt wird, ermöglicht der Kontaktkörper auf einfache Weise im Innern des Augengewebes Schnitte und Schnittflächen äquidistant zum kontaktierten Bereich des Auges zu erzeugen. Wenn der Brennpunkt äquidistant zu einer ebenen Bewegungsfläche bewegt wird, wird vorzugsweise ein als Applanationskörper ausgebildeter Kontaktkörper verwendet, der so angeordnet ist, dass er im aufgesetzten Zustand den kontaktierten Bereich des Auges parallel zu der Bewegungsfläche applaniert. Wenn der Brennpunkt äquidistant zu einer konkaven bzw. konvexen Bewegungsfläche bewegt wird, wird vorzugsweise ein als Konturkörper ausgebildeter Kontaktkörper verwendet, der so ausgebildet und angeordnet ist, dass er im aufgesetzten Zustand den kontaktierten Bereich des Auges parallel zu der konkaven bzw. konvexen Bewegungsfläche formt.

In einer Ausführungsvariante ist der Kontaktkörper fest mit den Befestigungsmitteln verbunden, so dass er am zu behandelnden Auge fixiert ist.

In einer Ausführungsvariante ist der Kontaktkörper fest mit dem Lichtprojektor verbunden. Dadurch wird ein fester Bezug vom Lichtprojektor zum Kontaktkörper geschaffen, wobei der Kontaktkörper bei Bewegungen, vorzugsweise bei Translationen, zusammen mit dem Lichtprojektor bewegt wird.

In einer Ausführungsvariante umfasst die ophthalmologische Vorrichtung eine Aufnahmevorrichtung zur entfernbaren Aufnahme des Kontaktkörpers. Dadurch können zur Wahrung der Hygiene und zur Vermeidung von Infektionen wegwerfbare Kontaktkörper verwendet werden. Dieser Vorteil wird insbesondere dann erreicht, wenn der Kontaktkörper fest mit den Befestigungsmitteln verbunden ist und sowohl der Kontaktkörper als auch die Befestigungsmittel über die Aufnahmevorrichtung entfernbar mit der ophthalmologischen Vorrichtung verbindbar sind und nach der Verwendung an einem Patienten weggeworfen oder sterilisiert werden können.

In einer Ausführungsvariante umfasst die Lichtquelle einen Femtosekundenlaser. Ein Femtosekundenlaser umfasst einen Lasergenerator zur Erzeugung von Laserpulsen mit Pulsbreiten von 1fs bis 1ps (1fs=10⁻¹⁵s). Laserpulse mit solchen Pulsbreiten ermöglichen die gezielte punktuelle Auflösung von Gewebe im Innern des Augengewebes, wobei mechanische und thermische Nebeneffekte im umgebenden Gewebe, wie man sie von längeren Pulsbreiten kennt, stark reduziert werden.

In einer Ausführungsvariante umfasst die Lichtquelle einen Femtosekundenlaser, der als Faserlaser ausgeführt ist. Ein Femtosekundenfaserlaser kann Platz sparend aufgewickelt werden, so dass die ophthalmologische Vorrichtung als in sich geschlossene Einheit ausführbar ist, welche die Lichtquelle und den mit der Lichtquelle optisch verbundenen Lichtprojektor umfasst und welche manuell applizierbar und am Auge fixierbar ist.

In einer Ausführungsvariante umfasst die Lichtquelle einen optischen Oszillator, und der optische Oszillator ist über einen optischen Expander, über eine optische Transportleitung und über einen optischen Kompressor mit dem Lichtprojektor verbunden, wobei die optische Transportleitung einen optischen Verstärker umfasst. Durch den optischen Expander wird ein mittels des optischen Oszillators erzeugter kurzer Femtosekundenlaserpuls gedehnt, beispielsweise um den Faktor 10⁴. Der gedehnte Laserpuls ist von geringer Intensität und lässt sich durch den optischen Verstärker auf eine grössere Amplitude verstärken. Durch den optischen Kompressor wird der verstärkte, gedehnte Laserpuls komprimiert und dadurch die Intensität des Laserpulses auf ein Mehrfaches des Spitzenwerts, auf den der optische Verstärker ausgelegt ist, beispielsweise um das 10⁴fache. Mit einem klein dimensionierten optischen Verstärker können so Laserpulse mit grosser Intensität erzeugt werden.

In einer Ausführungsvariante ist die optische Transportleitung als photonischer Kristall ausgeführt. Die Ausführung der optischen Transportleitung als photonischer Kristall, so genannte Photonic Cristal Fiber, ermöglicht die Übertragungseigenschaften gegenüber herkömmlichen Faserleitungen wesentlich zu verbessern, so dass hohe Leistungen übertragbar sind und dass die Laserpulse durch Dispersion nicht "verwaschen" werden.

In einer Ausführungsvariante umfasst die ophthalmologische Vorrichtung Tragemittel, um die ophthalmologische Vorrichtung beweglich an einem zur ophthalmologischen Vorrichtung externen Objekt zu befestigen, zum Beispiel an einem Eximerlaser. Solche Tragemittel ermöglichen es, das Gewicht der ophthalmologischen Vorrichtung auf das externe Objekt zu übertragen und das zu behandelnde Auge zu entlasten.

In einer Ausführungsvariante ist die ophthalmologische Vorrichtung eingerichtet, den Brennpunkt so zu bewegen, dass durch die Gewebeauflösung im Innern des Augengewebes ein Gewebelappen, der in einem Restbereich mit dem Auge verbunden bleibt, vom verbleibenden Augengewebe getrennt wird.

In einer Ausführungsvariante umfasst die ophthalmologische Vorrichtung Steuermittel zum Steuern der Position des Brennpunkts derart, dass durch die Gewebeauflösung im Innern des Augengewebes ein Gewebestück, das in einem Restbereich mit dem Auge verbunden bleibt, vom verbleibenden Augengewebe getrennt wird, wobei der Gewebelappen Seitenflächen aufweist, die durch Verändern des Abstands des Brennpunkts von der Bewegungsfläche erzeugt wird.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Vorrichtung darstellt, die einen Handgriff, Befestigungsmittel, eine Lichtquelle, einen mit der Lichtquelle optisch verbundenen Lichtprojektor sowie einen Bewegungstreiber zur Bewegung des Lichtprojektors umfasst.
Figur 2 zeigt ein Blockdiagramm, welches schematisch eine Ausführung der ophthalmologischen Vorrichtung mit einem auf das Auge aufsetzbaren, mindestens stellenweise lichtdurchlässigen Kontaktkörper darstellt, welcher fest mit den Befestigungsmitteln verbunden ist.
Figur 3 zeigt ein Blockdiagramm, welches schematisch eine Ausführung der ophthalmologischen Vorrichtung mit einem auf das Auge aufsetzbaren, mindestens stellenweise lichtdurchlässigen Kontaktkörper darstellt, welcher fest mit dem Lichtprojektor verbunden ist.
Figur 4a zeigt mit Bezug zu einem Auge die Kombination zweier translatorischer Bewegungen für die Bewegung des Brennpunkts des Lichtprojektors zur punktuellen Gewebeauflösung im Innern des Augengewebes.
Die Figuren 4b bis 4e zeigen mit Bezug zu einem Auge verschiedene Kombinationen einer translatorischen und einer rotatorischen Bewegung für die Bewegung des Brennpunkts des Lichtprojektors zur punktuellen Gewebeauflösung im Innern des Augengewebes.
Figur 4f zeigt mit Bezug zu einem Auge die Kombination zweier rotatorischer Bewegungen für die Bewegung des Brennpunkts des Lichtprojektors zur punktuellen Gewebeauflösung im Innern des Augengewebes.
Figur 5a zeigt ein Blockdiagramm, welches schematisch eine Ausführung der ophthalmologischen Vorrichtung mit lichtumlenkenden optischen Elementen zur Verschiebung des Brennpunkts entlang einer Scanlinie in einer Seitenansicht darstellt.
Figur 5b zeigt ein Blockdiagramm, welches schematisch eine Ausführung der ophthalmologischen Vorrichtung mit einem Bewegungstreiber zur Translation des Lichtprojektors entlang einer Translationsachse in einer Seitenansicht darstellt.
Figur 5c zeigt ein Blockdiagramm, welches schematisch eine Ausführung der ophthalmologischen Vorrichtung mit einem Bewegungstreiber zur Rotation des Lichtprojektors um eine Rotationsachse in einer Seitenansicht darstellt.
Figur 6a zeigt einen schematischen Querschnitt einer Ausführung der ophthalmologischen Vorrichtung mit einem ersten Bewegungstreiber zur Translation des Lichtprojektors entlang einer Translationsachse und mit einem zweiten Bewegungstreiber zur Rotation des Lichtprojektors um eine Rotationsachse.
Figur 6b zeigt einen Querschnitt, welcher schematisch eine Ausführung der Lagerung des Lichtprojektors für die Rotation des Lichtprojektors darstellt.
Figur 7 zeigt ein Blockdiagramm, welches schematisch eine Ausführung der ophthalmologischen Vorrichtung darstellt, die ein Handgerät und eine Lichtquelle umfasst, die über eine optische Transportleitung miteinander verbunden sind.
Figur 8 zeigt ein Blockdiagramm, welches schematisch eine Ausführung der ophthalmologischen Vorrichtung darstellt, die Tragemittel umfasst, um die ophthalmologische Vorrichtung beweglich an einem zur ophthalmologischen Vorrichtung externen Objekt zu befestigen.
Figur 9a zeigt einen Querschnitt eines Gewebelappens, der von Augengewebe getrennt in einem Restbereich mit dem Auge verbunden bleibt.
Figur 9b zeigt einen weiteren Querschnitt des in Figur 9a dargestellten Gewebelappens.
Figur 10a zeigt einen Querschnitt eines Gewebestücks, das von Augengewebe getrennt in einem Restbereich mit dem Auge verbunden bleibt und das Seitenflächen aufweist.
Figur 10b zeigt einen weiteren Querschnitt des in Figur 10a dargestellten Gewebestücks mit Seitenflächen.

### Wege zur Ausführung der Erfindung

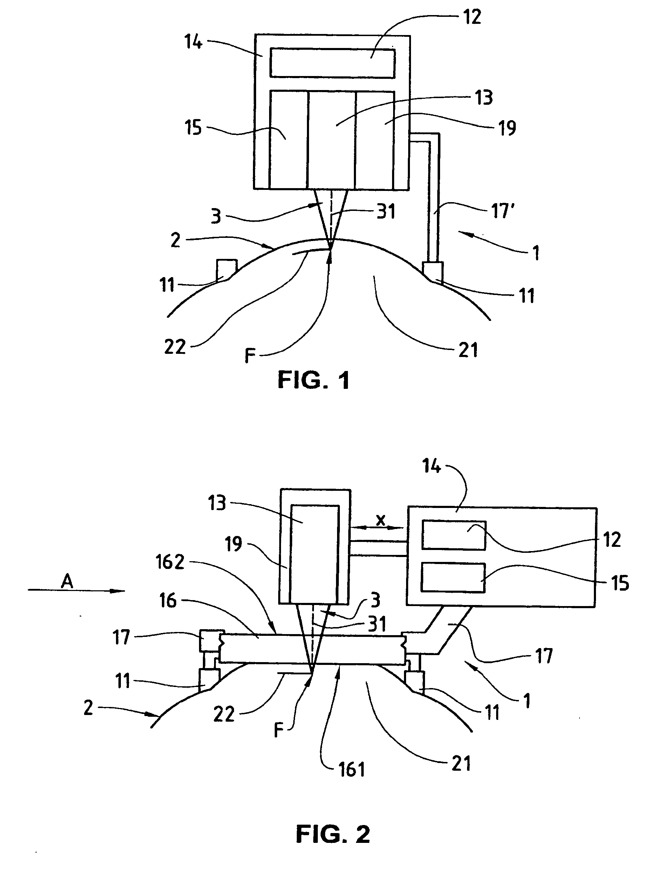

In den Figuren 1 bis 8 werden einander entsprechende Komponenten durch gleiche Bezugszeichen bezeichnet.

In den Figuren 1, 2, 3, 4a bis 4f, 5a bis 5c, 6a, 9a, 9b, 10a und 10b bezeichnet das Bezugszeichen 2 ein Auge, insbesondere ein menschliches Auge.

In den Figuren 1, 2, 3, 5a bis 5c, 6a, 9a, 9b, 10a und 10b bezeichnet das Bezugszeichen 21 Augengewebe, insbesondere Hornhautgewebe.

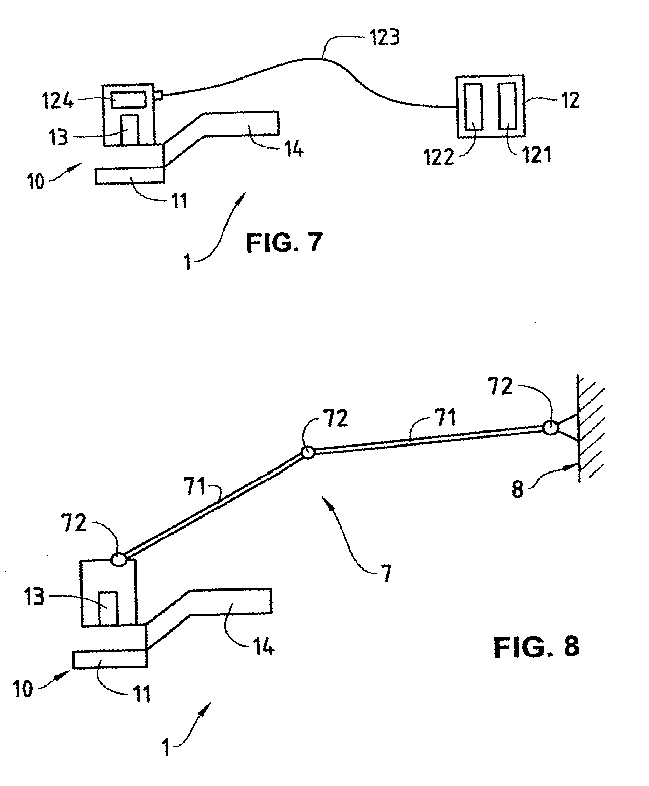

In den Figuren 1, 2, 3, 5a bis 5c, 6a, 7 und 8 bezeichnet das Bezugszeichen 1 eine ophthalmologische Vorrichtung, die einen Handgriff 14 zum manuellen Halten und Applizieren der ophthalmologischen Vorrichtung 1 umfasst. Wie in den Figuren 7 und 8 schematisch dargestellt ist, kann der Handgriff 14 von der ophthalmologischen Vorrichtung 1 vorstehend ausgeführt sein. Die ophthalmologische Vorrichtung 1 kann aber auch so als Handgerät ausgestaltet sein, dass ihre äussere Form als Handgriff 14 dient.

In den Figuren 1, 2, 3, 6a, 7 und 8 bezeichnet das Bezugszeichen 11 Befestigungsmittel 11 zum Fixieren der ophthalmologischen Vorrichtung 1 am Auge 2. Die Befestigungsmittel 11 umfassen beispielsweise einen Saugring oder mehrere Saugelemente, die die ophthalmologische Vorrichtung 1 durch Unterdruck am Auge 2 fixieren.

Die ophthalmologische Vorrichtung 1 umfasst einen Lichtprojektor 13 zur fokussierten Projektion von Lichtpulsen 3 für die punktuelle Gewebeauflösung an einem Brennpunkt F im Innern des Augengewebes 21. Der Lichtprojektor 13 ist optisch mit einer Lichtquelle 12 verbunden. Die Lichtquelle 12 umfasst einen Femtosekundenlaser, das heisst einen Lasergenerator zur Erzeugung von Laserpulsen mit Pulsbreiten im Femtosekundenbereich von 1fs bis 1ps (1fs=10⁻¹⁵S). Die Lichtquelle 12 umfasst beispielsweise einen Femtosekundenlaser, der als Faserlaser ausgeführt ist (Femtosekundenfaserlaser).

Wie in den Figuren 1, 2, und 3 dargestellt ist, ist die ophthalmologische Vorrichtung 1 als Einheit ausgeführt, die manuell applizierbar und am Auge 2 fixierbar ist und die die Lichtquelle 12 und den mit der Lichtquelle 12 optisch verbundenen Lichtprojektor 13 umfasst. Die Lichtquelle 12 umfasst beispielsweise einen entsprechend aufgewickelten Femtosekundenfaserlaser.

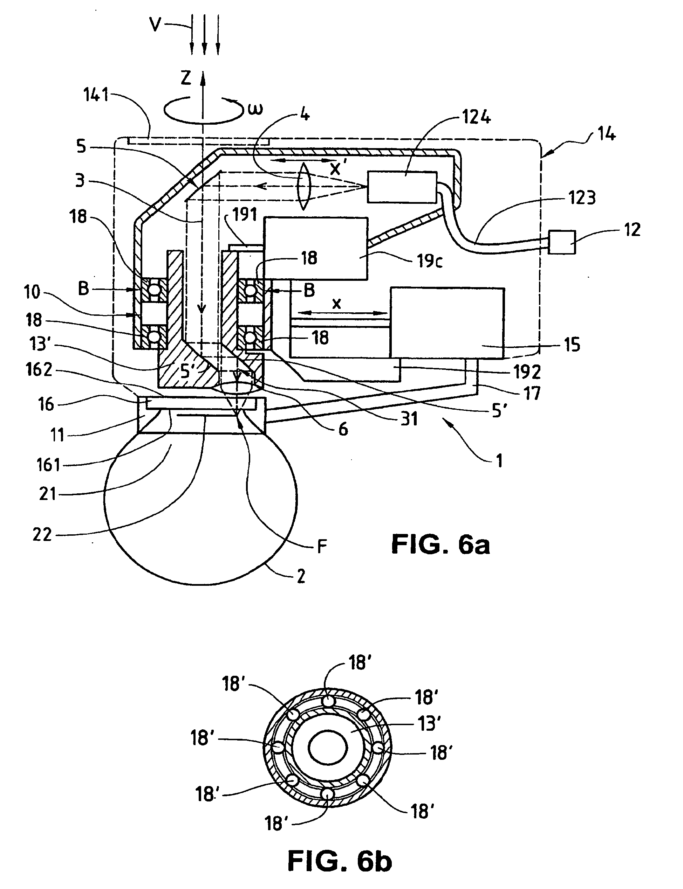

Wie in der Figur 7 dargestellt ist, umfasst die ophthalmologische Vorrichtung 1 in einer alternativen Ausführungsvariante ein Handgerät 10, das manuell applizierbar und am Auge 2 fixierbar ist und das einen Lichtprojektor 13 umfasst, sowie eine zum Handgerät 10 externe Lichtquelle 12, die mit dem Lichtprojektor 13 über eine optische Transportleitung 123 verbunden ist. Eine entsprechend ausgeführte ophthalmologische Vorrichtung 1 mit einem solchen Handgerät 10 ist auch in der Figur 6a schematisch dargestellt. Wie in der Figur 7 schematisch dargestellt ist, umfasst die Lichtquelle 12 beispielsweise einen optischen Oszillator 121, der über einen optischen Expander 122, über die optische Transportleitung 123 und über einen optischen Kompressor 124 mit dem Lichtprojektor 13 verbunden ist, wobei die optische Transportleitung 123 einen optischen Verstärker umfasst. Alternativ kann durch die Verwendung photonischer Kristalle hohe Leistung in das Handgerät 10 übertragen werden. Expander 122, Verstärker und auch der Kompressor 124 können dann extern vom Handgerät 10 liegen.

Wie in der Figur 8 dargestellt ist, kann die ophthalmologische Vorrichtung 1 in einer Ausführungsvariante auch als Handgerät 10 ausgeführt sein, das manuell applizierbar und am Auge 2 fixierbar ist und das mit Tragemitteln 7 versehen ist, um die ophthalmologische Vorrichtung 1 respektive das Handgerät 10 beweglich an einem zur ophthalmologischen Vorrichtung 1 respektive zum Handgerät 10 externen Objekt 8 zu befestigen. Die Tragemittel 7 umfassen beispielsweise mehrere Tragelemente 71, die über Gelenke 72 beweglich miteinander verbunden sind und die Gewichtskräfte aufnehmen können.

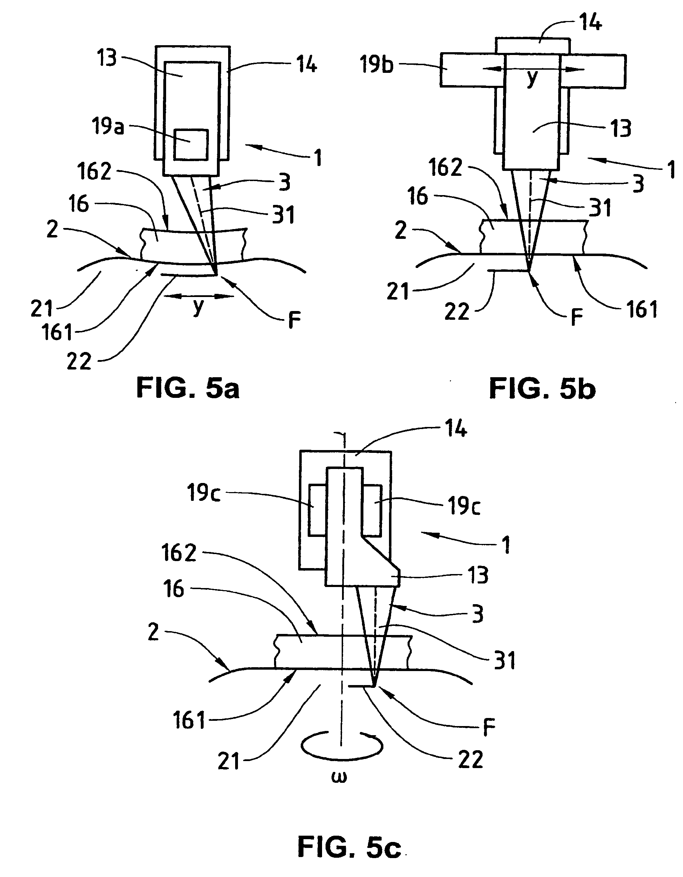

Wie in den Figuren 2, 3, 5a bis 5c, und 6a dargestellt ist, umfasst die ophthalmologische Vorrichtung 1 einen auf das Auge 2 aufsetzbaren, mindestens stellenweise lichtdurchlässigen Kontaktkörper 16. Der Kontaktkörper 16 besteht vorzugsweise aus transparentem Material, beispielsweise Kunststoff, mit einem dem Augengewebe 21 entsprechenden Brechungsindex. Der im aufgesetzten Zustand durch den Kontaktkörper 16 kontaktierte Bereich des Auges 2 wird als Kontaktbereich 161 bezeichnet. Der Kontaktkörper 16 weist eine vom Kontaktbereich 161 abgewandte Oberfläche 162 auf. Vorzugsweise ist der Kontaktkörper 16 als planparalleler Applanationskörper ausgestaltet und die Oberfläche 162 ist äquidistant zum Kontaktbereich 161 (siehe Figuren 2, 3, 5b, 5c und 6a). Der Kontaktkörper 16 kann jedoch auch als Konturkörper ausgestaltet sein, der den kontaktierten Bereich des Auges 2 beispielsweise konkav bzw. konvex verformt (siehe Figur 5a). Sowohl der als Applanationskörper als auch der als Konturkörper ausgestaltete Kontaktkörper 16 kann so ausgebildet sein, dass die Oberfläche 162 nicht äquidistant zum Kontaktbereich 161 ist. Der Kontaktkörper 16 kann in einer alternativen Ausführungsvariante auch eine Durchbrechung aufweisen, durch die die Laserpulse projiziert werden.

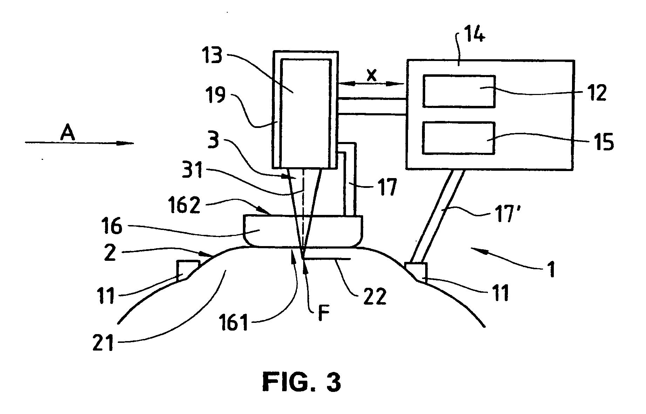

Vorzugsweise ist der Kontaktkörper 16 fest mit den Befestigungsmitteln 11 verbunden, wie in den Figuren 2 und 6a dargestellt ist. In einer alternativen Ausführung ist der Kontaktkörper 16 fest mit dem Lichtprojektor 13 verbunden, wie in der Figur 3 dargestellt ist. Dadurch ergibt sich der Vorteil, dass der Kontaktkörper 16 und der Lichtprojektor 13 präzise zueinander justiert sind.

Wie in den Figuren 2, 3 und 6a schematisch dargestellt ist, umfasst die ophthalmologische Vorrichtung 1 vorzugsweise eine Aufnahmevorrichtung 17 zur entfernbaren Aufnahme des Kontaktkörpers 16. Insbesondere, wenn der Kontaktkörper 16 fest mit den Befestigungsmitteln 11 verbunden ist, wie in den Figuren 2 und 6a dargestellt ist, ermöglicht die Aufnahmevorrichtung 17 die Entfernung des Kontaktkörpers 16 und der damit verbundenen Befestigungsmittel 11, nach der Applikation der ophthalmologischen Vorrichtung 1 auf das Auge 2 eines Patienten. Wie in den Figuren 1 und 3 dargestellt ist, kann auch eine entsprechende Aufnahmevorrichtung 17' zur entfernbaren Aufnahme der Befestigungsmittel 11 vorgesehen werden, falls diese nicht fest mit dem Kontaktkörper 16 verbunden sind. Die entfernten Kontaktkörper 16 und/oder Befestigungsmittel 11 können entweder weggeworfen oder gereinigt und sterilisiert werden. Die Aufnahmevorrichtung 17 respektive 17' kann beispielsweise so ausgestaltet werden, dass der Kontaktkörper 16 respektive die Befestigungsmittel 11 mittels eines Schraubverschlusses oder mittels eines Schnappverschlusses entfernbar mit der ophthalmologischen Vorrichtung 1 verbunden werden.

Sowohl die einfache Ausführungsvariante der ophthalmologischen Vorrichtung 1 ohne Kontaktkörper 16, die in der Figur 1 dargestellt ist, als auch die bevorzugte Ausführungsvariante, in der ein Kontaktkörper 16 fest mit den Befestigungsmitteln 11 verbunden ist, wovon ein Beispiel in der Figur 2 dargestellt ist, und eine weitere Ausführungsvariante, in der ein Kontaktkörper 16 fest mit dem Lichtprojektor 13 verbunden ist, wovon ein Beispiel in der Figur 3 dargestellt ist, verfügen über einen Bewegungstreiber 15 und weitere Bewegungsmittel 19, um den Brennpunkt F der vom Lichtprojektor 13 projizierten Lichtpulse 3 so zu bewegen, dass innerhalb des Augengewebes 21 Gewebeschnitte 22 gemacht werden können. Der Bewegungstreiber 15 ist eingerichtet und angeordnet, um den Lichtprojektor 13 und damit den Brennpunkt F mechanisch äquidistant zu einer Bewegungsfläche zu bewegen.

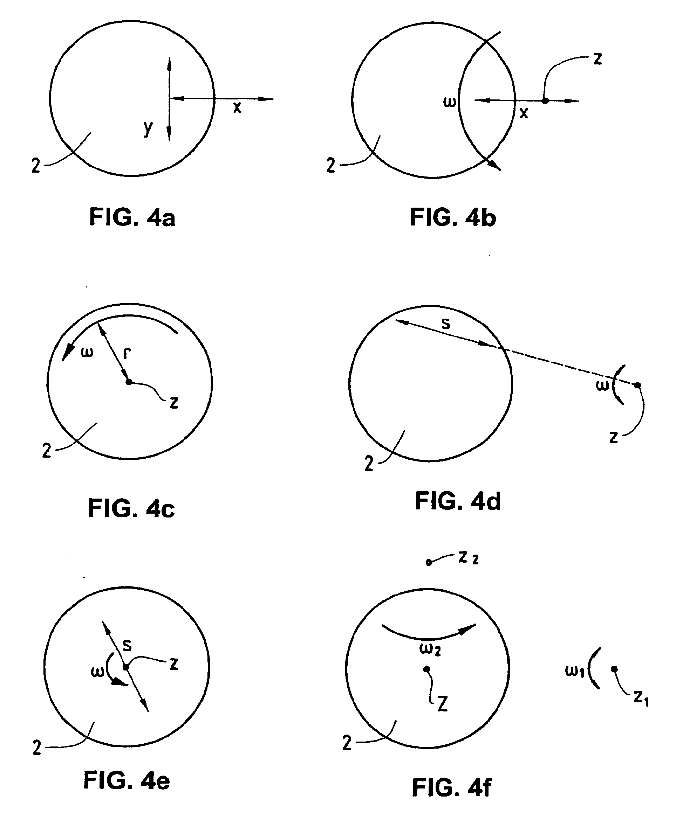

Die Figuren 4a bis 4f zeigen jeweils eine Aufsicht auf das Auge 2, wobei die Zeichnungsebene vorzugsweise parallel zu der Bewegungsfläche ist, zu welcher der Lichtprojektor 13 äquidistant bewegbar ist. In einer Ausführungsvariante ist der Lichtprojektor 13 äquidistant zu einer nicht ebenen Bewegungsfläche bewegbar. Die in den Figuren 4a bis 4f dargestellten Kombinationen von translatorischen und/oder rotatorischen Bewegungen für die Bewegung des Brennpunkts F im Innern des Augengewebes 21 können jeweils mit der erwähnten einfachen, mit der erwähnten bevorzugten und mit der erwähnten weiteren Ausführungsvariante der ophthalmologischen Vorrichtung 1 kombiniert werden. Um die in den Figuren 4a bis 4f dargestellten Kombinationen von translatorischen und/oder rotatorischen Bewegungen auszuführen, umfassen die Bewegungsmittel 19 einen weiteren Bewegungstreiber 19b, 19c oder bewegliche lichtumlenkende optische Elemente 19a wie nachfolgend beschrieben wird.

In der Figur 4a ist die Kombination zweier translatorischer Bewegungen für die Bewegung des Brennpunkts F dargestellt, die einer Scanoperation entspricht. Mindestens eine der Translationen wird durch einen Bewegungstreiber 15 bewirkt, der den Lichtprojektor 13, beispielsweise in der x-Richtung mechanisch bewegt. Entsprechende Ausführungen mit einem Bewegungstreiber 15 für eine Translation des Lichtprojektors 13 in der x-Richtung sind in den Figuren 2, 3 und 6a dargestellt. Die zweite Translation wird entweder durch einen weiteren Bewegungstreiber 19b bewirkt (siehe Figur 5b), der den Lichtprojektor 13 mechanisch in der y-Richtung bewegt, oder durch bewegliche lichtumlenkende optische Elemente 19a (siehe Figur 5a), die die Lichtpulse 3 entlang einer Scanlinie zur Ausführung des Gewebeschnitts 22 in der y-Richtung projizieren. Bewegliche optische Elemente 19a können beispielsweise als Spiegelpolygone oder Galvanoscanner realisiert werden. In einer Ausführungsvariante werden den translatorischen Bewegungen des Brennpunkts F in der x-Richtung und in der y-Richtung zusätzlich eine feine Bewegung des Brennpunkts F überlagert, beispielsweise mittels optischen Microscans.

In der Figur 4b ist eine Kombination einer translatorischen Bewegung und einer rotatorischen Bewegung für die Bewegung des Brennpunkts F dargestellt, die ähnlich einer Fräsoperation ist, wobei allerdings nur ein Schnitt im Innern des Augengewebes 21 erzeugt wird. Die Rotation wird entweder durch bewegliche lichtumlenkende optische Elemente bewirkt, die die Lichtpulse 3 entlang dem Drehpfeil w projizieren, oder durch einen Bewegungstreiber 19c (siehe Figuren 5c oder 6a), der den Lichtprojektor 13 um die Rotationsachse z rotiert, d.h. ein Rotationstreiber. Die Translation wird durch einen Bewegungstreiber 15 bewirkt, der die Rotationsachse z in der x-Richtung mechanisch bewegt (siehe insbesondere Figur 6a).

In der Figur 4c ist eine weitere Kombination einer translatorischen Bewegung und einer rotatorischen Bewegung für die Bewegung des Brennpunkts F dargestellt, die einer Spiralbewegung wie bei einer Schallplatte oder einer Compact Disk entspricht oder auch in Form von konzentrischen Kreisen ausgeführt werden kann. Die Rotation wird durch bewegliche lichtumlenkende optische Elemente bewirkt, die die Lichtpulse 3 entlang dem Drehpfeil w projizieren. Die Translation wird durch einen Bewegungstreiber 15 bewirkt, der eine radiale Translation des Lichtprojektors 13 auf dem von der Rotationsachse z ausgehenden Radius r ausführt.

In der Figur 4d ist eine weitere Kombination einer translatorischen Bewegung und einer rotatorischen Bewegung für die Bewegung des Brennpunkts F dargestellt, die der Bewegung eines Scheibenwischers bei einem Auto entspricht. Die Rotation wird durch einen Bewegungstreiber bewirkt, der den Lichtprojektor 13 entsprechend dem Drehpfeil w um die Rotationsachse z hin-und herschwenkt. Die Translation wird durch bewegliche lichtumlenkende optische Elemente bewirkt, die die Lichtpulse 3 entlang einer durch die Rotationsachse z verlaufenden Scanlinie s projizieren.

In der Figur 4e ist eine weitere Kombination einer translatorischen Bewegung und einer rotatorischen Bewegung für die Bewegung des Brennpunkts F dargestellt. Die Rotation wird durch einen Bewegungstreiber 19c (siehe Figuren 5c oder 6a) bewirkt, der den Lichtprojektor 13 um die Rotationsachse z rotiert, d.h. ein Rotationstreiber. Die Translation wird durch bewegliche lichtumlenkende optische Elemente bewirkt, die die Lichtpulse 3 entlang einer durch die Rotationsachse z verlaufenden Scanlinie s projizieren.

In der Figur 4f ist die Kombination zweier rotatorischer Bewegungen für die Bewegung des Brennpunkts F dargestellt. Durch einen ersten Bewegungstreiber wird der Lichtprojektor 13 entsprechend dem Drehpfeil w₁ um die Rotationsachse z₁ hin- und hergeschwenkt. Die zweite Rotation wird entweder durch einen zweiten Bewegungstreiber bewirkt, der den Lichtprojektor 13 entsprechend dem Drehpfeil w₂ um die Rotationsachse z₂ rotiert oder durch bewegliche lichtumlenkende optische Elemente, die die Lichtpulse 3 entlang dem Drehpfeil w₂ projizieren. Die Rotationsachse z₁ kann auch durch das Zentrum Z verlaufen.

Zu den oben beschriebenen Kombinationen von translatorischen und/oder rotatorischen Bewegungen soll hier bemerkt werden, dass die durch bewegliche lichtumlenkende optische Elemente ausgeführten Bewegungen des Brennpunkts F viel schneller ausgeführt werden (Oszillationsfrequenzen grösser 100 Hz sind technisch leicht realisierbar) als die durch mechanische Bewegungstreiber bewirkten Bewegungen des Brennpunkts F. Schnelle mechanische Scanbewegungen lassen sich nur durch Rotationen erzeugen, da dort keine Trägheitskräfte von oszillierenden Massen überwunden werden müssen. Durch eine Kombination von schnellen Bewegungen entlang einer Scanlinie und langsamen Translationsbewegungen senkrecht zur Scanlinie, lassen sich Schnittflächen genau so schnell erzeugen, wie wenn auch in senkrechter Richtung zur Scanlinie schnelles Scannen möglich wäre. Durch eine geeignete Einschränkung der Flexibilität, das heisst durch eine Begrenzung der möglichen Scanmuster, lässt sich so vorteilhaft der gerätetechnische Aufwand reduzieren (d.h. Wegfall einer schnellen optischen Scanachse). Weiterhin kann die Beschränkung der Flexibilität zur Reduzierung der Baugrösse der ophthalmologischen Vorrichtung 1 ausgenützt werden.

Wie in der Figur 2 dargestellt ist, ist der Bewegungstreiber 15 in der bevorzugten Ausführungsvariante der ophthalmologischen Vorrichtung 1 so ausgebildet und angeordnet, dass der Lichtprojektor 13 in der x-Richtung verschoben wird, die in der Zeichnungsebene verläuft. Der Lichtprojektor 13 wird durch den Bewegungstreiber 15 insbesondere entlang einer Translationslinie, respektive Translationsachse, in x-Richtung äquidistant zu der Bewegungsfläche verschoben. In der Figur 2 enthält die Bewegungsfläche beispielsweise die x-Achse und ist senkrecht zu der Zeichnungsebene. Der Kontaktkörper 16 ist vorzugsweise so angeordnet, dass der Kontaktbereich 161 äquidistant zu der Bewegungsfläche ist. Dadurch wird im Innern des Augengewebes 21 bei unveränderter Brennweite des Lichtprojektors 13, d.h. bei unveränderter Distanz vom Lichtprojektor 13 zum Brennpunkt F, ein Gewebeschnitt 22 äquidistant zu der Bewegungsfläche in x-Richtung erzeugt. Das Gleiche gilt auch für die in der Figur 3 dargestellte weitere Ausführungsvariante der ophthalmologischen Vorrichtung 1, in der, im Unterschied zur bevorzugten Ausführungsvariante, der Kontaktkörper 16 fest mit dem Lichtprojektor 13 verbunden ist. Wie aus den Figuren 2 und 3 ersichtlich ist, sind der Bewegungstreiber 15 und der Lichtprojektor 13 so gekoppelt und dimensioniert, dass der Lichtprojektor 13 in eine Position bewegbar ist, in welcher beim Fixieren der ophthalmologischen Vorrichtung 1 am Auge 2 (bei deaktivierter Lichtquelle 12) mindestens ein Teil des Auges 2 in einer Aufsicht für den Benutzer einsehbar ist. Dadurch lässt sich die ophthalmologische Vorrichtung 1 einfach und genau applizieren, beispielsweise wie ein herkömmliches Mikrokeratom. Der Vorgang lässt sich wie gewohnt durch das Operationsmikroskop verfolgen.

Wie in den Figuren 2 und 3 schematisch dargestellt ist, umfasst die ophthalmologische Vorrichtung 1 weitere Bewegungsmittel 19 für die Bewegung des Brennpunkts F in der zur x-Richtung senkrechten y-Richtung.

In den Figuren 5a, 5b und 5c sind verschiedene mit den in den Figuren 2 und 3 dargestellten Ausführungsvarianten kombinierbare Ausführungen der ophthalmologischen Vorrichtung 1 mit Bewegungsmitteln 19 für die Bewegung des Brennpunkts F in der zur x-Richtung senkrechten y-Richtung dargestellt. Die Figuren 5a, 5b und 5c zeigen schematische eine Ausschnittansicht der ophthalmologischen Vorrichtung 1 in der in den Figuren 2 und 3 durch den Pfeil A angezeigten Richtung.

In der Ausführungsvariante gemäss der Figur 5a umfasst die ophthalmologische Vorrichtung 1 bewegliche lichtumlenkende optische Elemente 19a, welche die Lichtpulse 3 zur Ausführung des Gewebeschnitts 22 in der y-Richtung auf eine Scanlinie projizieren, die äquidistant zu einer konkaven bzw. konvexen Bewegungsfläche verläuft. Der Kontaktkörper 16 ist vorzugsweise konkav bzw. konvex ausgebildet und so angeordnet, dass der Kontaktbereich 161 äquidistant zu der konkaven bzw. konvexen Bewegungsfläche ist. Dadurch wird im Innern des Augengewebes 21 bei unveränderter Brennweite des Lichtprojektors 13 ein Gewebeschnitt 22 äquidistant zu der konkaven bzw. konvexen Bewegungsfläche erzeugt. Die beweglichen lichtumlenkenden optischen Elemente 19a ermöglichen ein schnelles und wiederholtes Scannen in der y-Richtung (z.B. mit einer Frequenz von 100 bis 1000 Hz). Beispiele lichtumlenkender optischer Elemente 19a umfassen beispielsweise Polygonscanner, Galvanoscanner, Kippspiegel (über Piezo-Aktuatoren angesteuert), mikrooptoelektromechanische Systeme (MicroOptoElectroMechanical Systems, MOEMS), akustooptische Modulatoren (AOM) oder Mikrolinsen-Array-Scanner, die beispielsweise durch entsprechend programmierte Steuermittel gesteuert werden. Die Integration von anamorphotischen Optiken (z.B. Zylinderlinsen) entlang der Scanlinie ermöglicht hohe numerische Aperturen quer zur Scanrichtung.

In der Ausführungsvariante gemäss der Figur 5b umfasst die ophthalmologische Vorrichtung 1 einen weiteren Bewegungstreiber 19b, der so angeordnet ist, dass der Lichtprojektor 13 äquidistant zu einer ebenen Bewegungsfläche mechanisch entlang einer Translationsachse in der y-Richtung verschoben wird. Der Kontaktkörper 16 ist wiederum so angeordnet, dass der Kontaktbereich 161 äquidistant zu der Bewegungsfläche ist. Dadurch wird im Innern des Augengewebes 21 bei unveränderter Brennweite des Lichtprojektors 13 ein Gewebeschnitt 22 äquidistant zu der ebenen Bewegungsfläche in y-Richtung erzeugt.

In der Ausführungsvariante gemäss der Figur 5c umfasst die ophthalmologische Vorrichtung 1 einen weiteren Bewegungstreiber 19c, der so ausgebildet und angeordnet ist, dass der Lichtprojektor 13 entsprechend dem Drehpfeil ω um eine oder zwei zu einer ebenen Bewegungsfläche normalen Rotationsachse(n) z rotiert wird. Der Kontaktkörper 16 ist wiederum so angeordnet, dass der Kontaktbereich 161 äquidistant zu der Bewegungsfläche ist. Dadurch wird im Innern des Augengewebes 21 bei unveränderter Brennweite des Lichtprojektors 13 ein kreisförmiger Gewebeschnitt 22 äquidistant zu der ebenen Bewegungsfläche erzeugt.

Um den Brennpunkt F in der zur x- und y-Richtung senkrechten z-Richtung, die in den Figuren 5c und 6a angezeigt wird, zu bewegen, ist die ophthalmologische Vorrichtung 1 eingerichtet, den Abstand zwischen dem Brennpunkt F und der Bewegungsfläche zu regeln. In einer Ausführungsvariante umfassen die Bewegungsmittel 19 einen weiteren nicht dargestellten Bewegungstreiber, der den Lichtprojektor 13 in der z-Richtung bewegt, um den Abstand zwischen dem Brennpunkt F und der Bewegungsfläche zu verändern. Vorzugsweise ist die ophthalmologische Vorrichtung 1 jedoch eingerichtet, die Position des Brennpunkts F entlang der Projektionsachse 31 der Lichtpulse 3 zu regeln, d.h. die Lage des Brennpunkts F mittels optischer Elemente zu verändern. Die Brennweite des Lichtprojektors 13 kann beispielsweise durch Bewegen der Linse 4 in der x'-Richtung verändert werden, wie in der Figur 6a gezeigt wird. Durch Bewegen des Brennpunkts F in der z-Richtung können Gewebeschnitte im Innern des Augengewebes 21 in der z-Richtung, vertikal zur Bewegungsfläche, erzeugt werden.

In der Figur 6a wird ein schematischer Querschnitt einer Ausführung der ophthalmologischen Vorrichtung 1 im auf dem Auge 2 aufgesetzten Zustand gezeigt. Die in der Figur 6a gezeigte Ausführung der ophthalmologischen Vorrichtung 1 entspricht einer Kombination der in den Figuren 2 und 5c gezeigten Ausführungsvarianten und ermöglicht Bewegungen des Brennpunkts F gemäss der Figur 4b. Die in der Figur 6a gezeigte Ausführung der ophthalmologischen Vorrichtung 1 umfasst ein Handgerät 10 mit einem Lichtprojektor, der über eine optische Transportleitung 123 mit einer zum Handgerät 10 externen Lichtquelle 12 verbunden ist. Der Lichtprojektor gemäss der in der Figur 6a dargestellten Ausführung umfasst den Hohlkörper 13', die am Hohlkörper 13' angebrachten Spiegel 5' und Linse 6, sowie die Linse 4 und den Spiegel 5. In der Lichtquelle 12 generierte Lichtpulse 3 werden über die Linse 4, die Spiegel 5 und 5' und über die Linse 6 im Brennpunkt F fokussiert in das Innere des Augengewebes 21 projiziert. Der Hohlkörper 13' ist mittels der Lager 18 um die Rotationsachse z drehbar gelagert. Die Figur 6b zeigt einen Querschnitt durch das Lager 18 und den Hohlkörper 13' des Lichtprojektors an der Stelle, die in der Figur 6a durch die Pfeile B angezeigt ist, wobei die Anordnung von Lagerkugeln 18' bloss schematisch dargestellt wird. Der Fachmann wird verstehen, dass andere Lagerungsarten möglich sind. Der Hohlkörper 13' des Lichtprojektors wird, durch den Bewegungstreiber 19c über das Antriebselement 191 angetrieben, um die Rotationsachse z rotiert. Der Spiegel 5 ist starr mit dem Bewegungstreiber 19c verbunden und spiegelt einkommende Lichtpulse 3 auf die Spiegel 5', die fest mit dem drehbaren Hohlkörper 13' des Lichtprojektors verbunden sind und sich mit dem Hohlkörper 13' um die Rotationsachse z bewegen. Der Hohlkörper 13' des Lichtprojektors, die Lager 18 und der Bewegungstreiber 19c sind an einem Träger 192 angebracht, der durch den Bewegungstreiber 15 mechanisch entlang einer Translationsachse in der x-Richtung verschiebbar ist. Wie in der Figur 6a angedeutet ist, umfasst die ophthalmologische Vorrichtung 1 ein gestrichelt dargestelltes Gehäuse, das als Handgriff 14 dient und so ausgestaltet ist, dass die Translationsbewegung in der x-Richtung und die Rotation um die Rotationsachse z möglich ist. Das als Handgriff 14 ausgestaltete Gehäuse weist eine Aussparung 141 auf, die ein freie Aufsicht auf das Auge 2 aus der durch die Pfeile V angezeigten Richtung ermöglicht. Aus Figur 6a ist ersichtlich, wie durch die Kombination von mechanischen Scanbewegungen und kleinen Projektionsoptiken, eine kompakte ophthalmologische Vorrichtung 1 realisiert werden kann, die trotzdem eine sehr hohe numerische Apertur aufweist (die Ausführung gemäss Figur 6a weist ungefähr eine numerische Apertur von 0.5 auf).

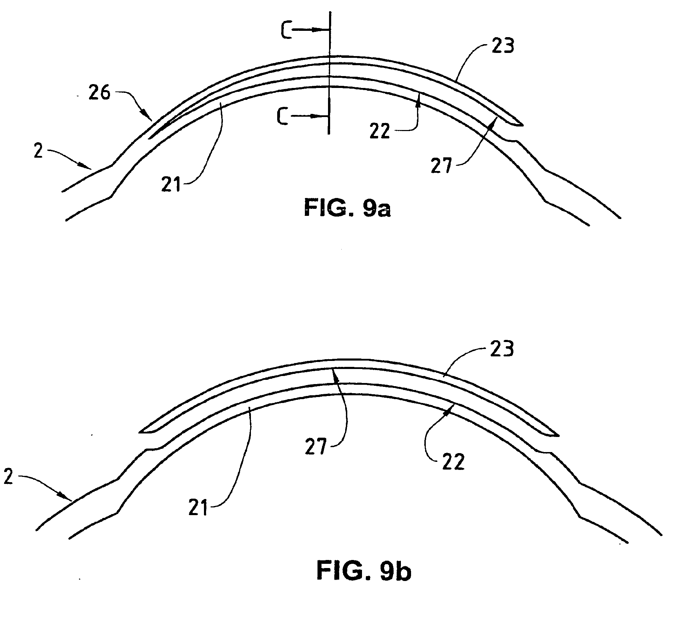

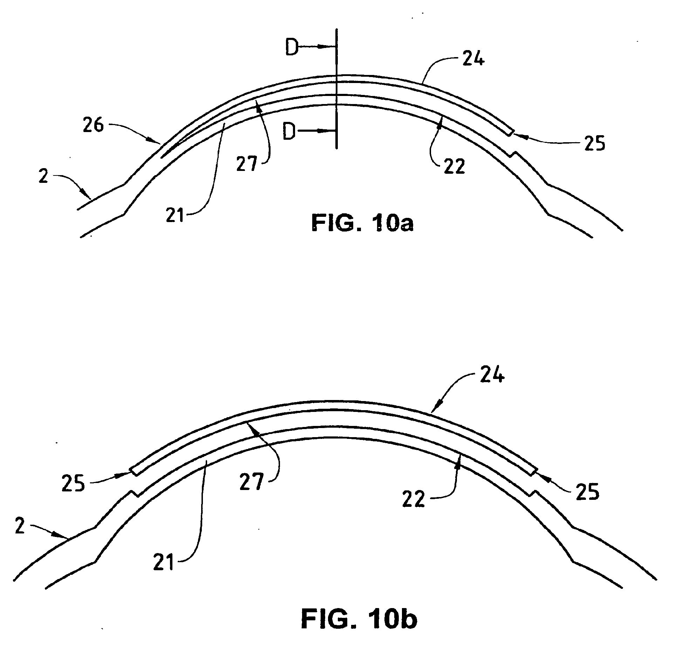

Die Figuren 9a, 9b, 10a und 10b zeigen Beispiele von Gewebestücken, die durch die ophthalmologische Vorrichtung 1 mittels fokussierter Projektion von Lichtpulsen auf Brennpunkte F im Innern des Augengewebes 21 und durch die dadurch bewirkte punktuelle Gewebeauflösung vom verbleibenden Augengewebe 21 getrennt werden. Der in der Figur 9a dargestellte Gewebelappen 23 wird im applanierten Zustand des Auges 2 durch einen ebenen Gewebeschnitt 22 im Innern des Augengewebes 21 erzeugt und bleibt in einem Restbereich 26 mit dem Auge 2 verbunden. Die Figur 9b zeigt einen Querschnitt durch den Gewebelappen 23 an der Stelle, die in der Figur 9a durch die Pfeile C angezeigt ist. Das in der Figur 10a gezeigte Gewebestück 24 wird auch im applanierten Zustand des Auges 2 durch einen ebenen Gewebeschnitt 22 im Innern des Augengewebes 21 erzeugt. Das Gewebestück 24 bleibt ebenfalls in einem Restbereich 26 mit dem Auge 2 verbunden und weist Seitenflächen 25 auf, die durch vertikale Schnitte erzeugt werden. Die Figur 10b zeigt einen Querschnitt durch das Gewebestück 24 mit den Seitenflächen 25 an der Stelle, die in der Figur 10a durch die Pfeile D angezeigt ist. Andere Kantenformen an Stelle der Seitenflächen 25 sind möglich, wenn beispielsweise Linse 4 in Figur 6a zusätzlich vertikal verschoben wird. Durch den ebenen Gewebeschnitt 22, das heisst durch Bewegung des Brennpunkts F äquidistant zu der Bewegungsfläche, werden die Grundflächen 27 des in der Figur 9a dargestellten Gewebelappens 23 und des in der Figur 10a gezeigten Gewebestücks 24 erzeugt. Um den Aktionsbereich, innerhalb dessen der Brennpunkt F bewegbar ist, zu begrenzen, sind der Bewegungstreiber 15 und die Bewegungsmittel 19, insbesondere 19a, 19b und 19c, mechanisch und/oder durch programmierbare Steuermittel in ihrem Bewegungsbereich begrenzt. Insbesondere für die Bewegung des Brennpunkts F äquidistant zu der Bewegungsfläche werden der Bewegungstreiber 15 und die Bewegungsmittel 19, insbesondere 19a, 19b und 19c, vorzugsweise mechanisch geführt. Durch mechanische Führungen lassen sich so Bewegungsflächen mit grosser Präzision realisieren. Insbesondere bei konstantem Arbeitsabstand des Lichtprojektors 13 können präzise Bewegungsflächen durch mechanische Führungen einfach realisiert werden. Programmierbare Steuermittel eignen sich insbesondere für die Konfiguration von individuell dimensionierten Gewebeschnitten 22 und/oder von Gewebeschnitten für das Trennen von Gewebestücken 24, die nicht bloss durch einfache ebene Gewebeschnitte 22 erzeugt werden können. Die programmierbaren Steuermittel umfassen beispielsweise einen Prozessor und einen Speicher mit darin gespeicherten Programmen und Schnittparametern, oder nicht programmierbare elektronische Schaltungen, die Speicher zur Speicherung von Schnittparametern umfassen.

Bei der Applikation der ophthalmologischen Vorrichtung 1 wird vorzugsweise zunächst der Lichtprojektor 13 mittels des Bewegungstreibers 15 so verschoben, dass mindestens ein Teil des Auges 2 in der Aufsicht frei einsehbar ist. Danach wird die ophthalmologische Vorrichtung 1 im gewünschten Bereich auf das Auge 2 aufgesetzt und mittels der Befestigungsmittel 11 am Auge 2 fixiert. Gegebenenfalls weist der Kontaktkörper 16 Referenzmarkierungen für eine genaue Positionierung der ophthalmologischen Vorrichtung 1 auf dem Auge auf. Dann wird die ophthalmologische Vorrichtung 1 aktiviert und der Brennpunkt F wird durch den Bewegungstreiber 15 und die Bewegungsmittel 19, insbesondere 19a, 19b und 19c, vorzugsweise unter mechanischer Führung und Begrenzung und/oder unter programmierter Steuerung automatisch bewegt.

## Patentansprüche

1. Ophthalmologische Vorrichtung (1), umfassend:
einen Handgriff (14) zum manuellen Halten und Applizieren der ophthalmologischen Vorrichtung (1),
Befestigungsmittel (11) zum Fixieren der ophthalmologischen Vorrichtung (1) an einem Auge (2), und
eine Lichtquelle (12) und einen mit der Lichtquelle (12) optisch verbundenen Lichtprojektor (13) zur fokussierten Projektion von Lichtpulsen (3) für die punktuelle Gewebeauflösung an einem Brennpunkt (F) im Innern des Augengewebes (21),
**gekennzeichnet durch,**
einen Bewegungstreiber (15) zur translatorischen Bewegung des Lichtprojektors und **dadurch** des Brennpunkts (F) in einer x-Richtung,
Bewegungsmittel (19, 19a, 19b) zur translatorischen Bewegung des Brennpunkts (F) in einer zur x-Richtung senkrechten y-Richtung, und
Mittel zur Überlagerung einer zusätzlichen feinen Bewegung des Brennpunkts (F) zu den translatorischen Bewegungen des Brennpunkts (F).

2. Ophthalmologische Vorrichtung (1) nach Anspruch 1, **dadurch gekennzeichnet, dass** die Mittel zur Überlagerung der zusätzlichen Bewegung des Brennpunkts (F) eingerichtet sind die Überlagerung der zusätzlichen Bewegung des Brennpunkts (F) mittels optischer Microscans durchzuführen.

3. Ophthalmologische Vorrichtung (1) nach einem der Ansprüche 1 oder 2, **dadurch gekennzeichnet, dass** der Bewegungstreiber (15) und der Lichtprojektor (13) so eingerichtet und gekoppelt sind, dass der Lichtprojektor (13) in eine Position bewegbar ist, in welcher beim Fixieren der ophthalmologischen Vorrichtung (1) am Auge (2) mindestens ein Teil des Auges (2) in einer Aufsicht einsehbar ist.

4. Ophthalmologische Vorrichtung (1) nach einem der Ansprüche 1 bis 3, **dadurch gekennzeichnet, dass** der Bewegungstreiber (15) und der Lichtprojektor (13) so eingerichtet und gekoppelt sind, dass der Lichtprojektor (13) äquidistant zu einer Bewegungsfläche bewegbar ist.

5. Ophthalmologische Vorrichtung (1) nach Anspruch 4, **dadurch gekennzeichnet, dass** der Bewegungstreiber (15) eingerichtet ist, den Lichtprojektor (13) entlang einer zur Bewegungsfläche äquidistanten Translationslinie zu bewegen.

6. Ophthalmologische Vorrichtung (1) nach einem der Ansprüche 4 oder 5, **dadurch gekennzeichnet, dass** sie eingerichtet ist, den Abstand zwischen dem Brennpunkt (F) und der Bewegungsfläche zu regeln.

7. Ophthalmologische Vorrichtung (1) nach einem der Ansprüche 1 bis 6, **dadurch gekennzeichnet, dass** sie eingerichtet ist, die Position des Brennpunkts (F) entlang der Projektionsachse (31) der Lichtpulse (3) zu regeln.

8. Ophthalmologische Vorrichtung (1) nach einem der Ansprüche 1 bis 7, **dadurch gekennzeichnet, dass** der Lichtprojektor (13) lichtumlenkende optische Elemente (19a) zur fokussierten Projektion der Lichtpulse (3) entlang einer Scanlinie umfasst.

9. Ophthalmologische Vorrichtung (1) nach einem der Ansprüche 4 bis 8, **dadurch gekennzeichnet, dass** sie einen auf das Auge (2) aufsetzbaren, mindestens stellenweise lichtdurchlässigen Kontaktkörper (16) umfasst, welcher so ausgebildet und angeordnet ist, dass er einen im aufgesetzten Zustand kontaktierten Bereich (161) des Auges (2) äquidistant zu der Bewegungsfläche setzt.

10. Ophthalmologische Vorrichtung (1) nach Anspruch 9, **dadurch gekennzeichnet, dass** der Kontaktkörper (16) planparallel ausgestaltet ist.

11. Ophthalmologische Vorrichtung (1) nach einem der Ansprüche 9 oder 10, **dadurch gekennzeichnet, dass** der Kontaktkörper (16) fest mit den Befestigungsmitteln (11) verbunden ist.

12. Ophthalmologische Vorrichtung (1) nach einem der Ansprüche 9 oder 10, **dadurch gekennzeichnet, dass** der Kontaktkörper (16) fest mit dem Lichtprojektor (13) verbunden ist.

13. Ophthalmologische Vorrichtung (1) nach einem der Ansprüche 9 bis 12, **dadurch gekennzeichnet, dass** sie eine Aufnahmevorrichtung (17) zur entfernbaren Aufnahme des Kontaktkörpers (16) umfasst.

14. Ophthalmologische Vorrichtung (1) nach einem der Ansprüche 1 bis 13, **dadurch gekennzeichnet, dass** der Bewegungstreiber (15) und der Lichtprojektor (13) so eingerichtet und gekoppelt sind, dass der Lichtprojektor (13) äquidistant zu einer ebenen Bewegungsfläche bewegbar ist.

15. Ophthalmologische Vorrichtung (1) nach einem der Ansprüche 1 bis 14, **dadurch gekennzeichnet, dass** die Lichtquelle (12) einen Femtosekundenlaser umfasst.

16. Ophthalmologische Vorrichtung (1) nach Anspruch 15, **dadurch gekennzeichnet, dass** der Femtosekundenlaser als Faserlaser ausgeführt ist.

17. Ophthalmologische Vorrichtung (1) nach einem der Ansprüche 1 bis 16, **dadurch gekennzeichnet, dass** die Lichtquelle (12) mit dem Lichtprojektor (13) über eine optische Transportleitung (123) verbunden ist, die als photonischer Kristall ausgeführt ist.

18. Ophthalmologische Vorrichtung (1) nach einem der Ansprüche 1 bis 16, **dadurch gekennzeichnet, dass** die Lichtquelle einen optischen Oszillator (121) umfasst, und dass der optische Oszillator (121) über einen optischen Expander (122), über eine optische Transportleitung (123) und über einen optischen Kompressor (124) mit dem Lichtprojektor (13) verbunden ist, wobei die optische Transportleitung (123) einen optischen Verstärker umfasst.

19. Ophthalmologische Vorrichtung (1) nach Anspruch 18, **dadurch gekennzeichnet, dass** die optische Transportleitung (123) als photonischer Kristall ausgeführt ist.

20. Ophthalmologische Vorrichtung (1) nach einem der Ansprüche 1 bis 19, **dadurch gekennzeichnet, dass** die Befestigungsmittel (11) eingerichtet sind, die ophthalmologische Vorrichtung (1) durch Unterdruck am Auge (2) zu fixieren.

21. Ophthalmologische Vorrichtung (1) nach einem der Ansprüche 1 bis 20, **dadurch gekennzeichnet, dass** sie Tragemittel (7) umfasst, um die ophthalmologische Vorrichtung (1) beweglich an einem zur ophthalmologischen Vorrichtung externen Objekt (8) zu befestigen.

22. Ophthalmologische Vorrichtung (1) nach einem der Ansprüche 1 bis 21, **dadurch gekennzeichnet, dass** sie eingerichtet ist, den Brennpunkt (F) so zu bewegen, dass durch die Gewebeauflösung im Innern des Augengewebes (21) ein Gewebelappen (23), der in einem Restbereich (26) mit dem Auge (2) verbunden bleibt, vom verbleibenden Augengewebe (21) getrennt wird.

## Claims

1. Ophthalmological apparatus (1), comprising:
a handle (14) for manually holding and applying the ophthalmological apparatus (1),
fastening means (11) for fixing the ophthalmological apparatus (1) at an eye (2), and
a light source (12) and a light projector (13), optically connected to the light source (12), for the focussed projection of light pulses (3) for punctiform tissue breakdown at a focal point (F) in the interior of the eye tissue (21),
**characterized by**
a movement driver (15) for moving the light projector and thereby the focal point (F) with a translatory movement in an x-direction,
movement means (19, 19a, 19b) for moving the focal point (F) with a translatory movement in a y-direction normal to the x-direction, and
means for superimposing an additional fine movement of the focal point (F) onto the translatory movements of the focal point (F).

2. Ophthalmological apparatus (1) according to Claim 1, **characterized in that** the means for superimposing the additional fine movement of the focal point (F) are configured to execute the superimposing of the additional fine movement of the focal point (F) by means of optical microscans.

3. Ophthalmological apparatus (1) according to one of Claims 1 or 2, **characterized in that** the movement driver (15) and the light projector (13) are set up and coupled such that the light projector (13) can be moved into a position in which at least a portion of the eye (2) can be examined in top view upon fixing the ophthalmological apparatus (1) at the eye (2).

4. Ophthalmological apparatus (1) according to one of Claims 1 to 3, **characterized in that** the movement driver (15) and the light projector (13) are set up and coupled such that the light projector (13) can be moved equidistantly from a movement surface.

5. Ophthalmological apparatus (1) according to Claim 4, **characterized in that** the movement driver (15) is set up to move the light projector (13) along a translation line equidistant from the movement surface.

6. Ophthalmological apparatus (1) according to one of Claims 4 or 5, **characterized in that** it is set up to control the spacing between the focal point (F) and the movement surface.

7. Ophthalmological apparatus (1) according to one of Claims 1 to 6, **characterized in that** it is set up to control the position of the focal point (F) along the axis of projection (31) of the light pulses (3).

8. Ophthalmological apparatus (1) according to one of Claims 1 to 7, **characterized in that** the light projector (13) comprises light-deflecting optical elements (19a) for the focussed projection of the light pulses (3) along a scanning line.

9. Ophthalmological apparatus (1) according to one of Claims 4 to 8, **characterized in that** it comprises a contact body (16) which can be mounted onto the eye (2) and is transparent at least at some points and is designed and arranged such that it places equidistantly from the movement surface an area (161) of the eye (2) which is contacted in the mounted state.

10. Ophthalmological apparatus (1) according to Claim 9, **characterized in that** the contact body (16) is of plane-parallel configuration.

11. Ophthalmological apparatus (1) according to one of Claims 9 or 10, **characterized in that** the contact body (16) is permanently connected to the fastening means (11).

12. Ophthalmological apparatus (1) according to one of Claims 9 or 10, **characterized in that** the contact body (16) is permanently connected to the light projector (13).

13. Ophthalmological apparatus (1) according to one of Claims 9 to 12, **characterized in that** it comprises a holding apparatus (17) for removably holding the contact body (16).

14. Ophthalmological apparatus (1) according to one of Claims 1 to 13, **characterized in that** the movement driver (15) and the light projector (13) are set up and coupled such that the light projector (13) can be moved equidistantly from a flat movement surface.

15. Ophthalmological apparatus (1) according to one of Claims 1 to 14, **characterized in that** the light source (12) comprises a femtosecond laser.

16. Ophthalmological apparatus (1) according to Claim 15, **characterized in that** the femtosecond laser is designed as a fibre laser.

17. Ophthalmological apparatus (1) according to one of Claims 1 to 16, **characterized in that** the light source (12) is connected to the light projector (13) via an optical transport line (123) which is designed as a photonic crystal.

18. Ophthalmological apparatus (1) according to one of Claims 1 to 16, **characterized in that** the light source comprises an optical oscillator (121), and **in that** the optical oscillator (121) is connected to the light projector (13) via an optical expander (122), via an optical transport line (123) and via an optical compressor (124), the optical transport line (123) comprising an optical amplifier.

19. Ophthalmological apparatus (1) according to Claim 18, **characterized in that** the optical transport line (123) is designed as a photonic crystal.

20. Ophthalmological apparatus (1) according to one of Claims 1 to 19, **characterized in that** the fastening means (11) are set up to fix the ophthalmological apparatus (1) by low pressure at the eye (2).

21. Ophthalmological apparatus (1) according to one of Claims 1 to 20, **characterized in that** it comprises carrying means (7) for fastening the ophthalmological apparatus (1) movably on an object (8) external to the ophthalmological apparatus.

22. Ophthalmological apparatus (1) according to one of Claims 1 to 21, **characterized in that** it is set up to move the focal point (F) such that the tissue breakdown in the interior of the eye tissue (21) separates from the remaining eye tissue (21) a tissue flap (23) which remains connected to the eye (2) in a residual area 26).

## Revendications

1. Dispositif ophtalmologique (1), comprenant :
une poignée (14) pour la fixation et l'application manuelles du dispositif ophtalmologique (1),
des moyens de fixation (11) pour la fixation du dispositif ophtalmologique (1) contre un oeil (2), et
une source de lumière (12) et un projecteur de lumière (13) connecté optiquement à la source de lumière (12) pour la projection focalisée d'impulsions lumineuses (3) pour l'ablation ponctuelle de tissu au niveau d'un foyer (F) à l'intérieur du tissu oculaire (21),
**caractérisé par**
un dispositif de déplacement (15) pour déplacer le projecteur de lumière et en conséquence du foyer (F) avec une mouvement de translation dans une direction x, moyens de déplacement (19, 19a, 19b) pour déplacer le foyer (F) avec une mouvement de translation dans une direction y normal à la direction x, et
moyens pour superposer une mouvement précis supplémentaire du foyer (F) aux mouvements de translation du foyer (F).

2. Dispositif ophtalmologique (1) selon la revendication 1, **caractérisé en ce que** les moyens pour superposer le mouvement supplémentaire du foyer (F) sont configurées pour effectuer la superposition de la mouvement supplémentaire du foyer (F) au moyen de microscans optiques.

3. Dispositif ophtalmologique (1) selon l'une quelconque des revendications 1 ou 2, **caractérisé en ce que** le dispositif de déplacement (15) et le projecteur de lumière (13) sont disposés et accouplés de telle sorte que le projecteur de lumière (13) puisse être déplacé dans une position dans laquelle, lors de la fixation du dispositif ophtalmologique (1) contre l'oeil (2), au moins une partie de l'oeil (2) puisse être vue dans un vue d'en haut.

4. Dispositif ophtalmologique (1) selon l'une quelconque des revendications 1 à 3, **caractérisé en ce que** le dispositif de déplacement (15, 19b) et le projecteur de lumière (13) sont disposés et accouplés de telle sorte que le projecteur de lumière (13) puisse être déplacé de manière équidistante par rapport à une surface de déplacement.

5. Dispositif ophtalmologique (1) selon la revendication 4, **caractérisé en ce que** le dispositif de déplacement (15, 19b) est disposé de manière à déplacer le projecteur de lumière (13) le long d'une ligne de translation équidistante par rapport à la surface de déplacement.

6. Dispositif ophtalmologique (1) selon l'une quelconque des revendications 4 ou 5, **caractérisé en ce qu'**il est disposé de manière à régler la distance entre le foyer (F) et la surface de déplacement.

7. Dispositif ophtalmologique (1) selon l'une quelconque des revendications 1 à 6, **caractérisé en ce qu'**il est disposé de manière à régler la position du foyer (F) le long de l'axe de projection (31) des impulsions lumineuses (3).

8. Dispositif ophtalmologique (1) selon l'une quelconque des revendications 1 à 7, **caractérisé en ce que** le projecteur de lumière (13) comprend des éléments optiques (19a) déviant la lumière pour la projection focalisée des impulsions lumineuses (3) le long d'une ligne de balayage.

9. Dispositif ophtalmologique (1) selon l'une quelconque des revendications 4 à 8, **caractérisé en ce qu'**il comprend un corps de contact (16) transparent au moins par endroits, pouvant être posé sur l'oeil (2), qui est réalisé et disposé de telle sorte qu'il place une région (161) de l'oeil (2) mise en contact dans l'état posé, à une distance équidistante de la surface de déplacement.

10. Dispositif ophtalmologique (1) selon la revendication 9, **caractérisé en ce que** le corps de contact (16) est réalisé sous forme plan-parallèle.

11. Dispositif ophtalmologique (1) selon l'une quelconque des revendications 9 ou 10, **caractérisé en ce que** le corps de contact (16) est connecté fixement aux moyens de fixation (11).

12. Dispositif ophtalmologique (1) selon l'une quelconque des revendications 9 ou 10, **caractérisé en ce que** le corps de contact (16) est connecté fixement au projecteur de lumière (13).

13. Dispositif ophtalmologique (1) selon l'une quelconque des revendications 9 à 12, **caractérisé en ce qu'**il comprend un dispositif de réception (17) pour recevoir le corps de contact (16) de manière à pouvoir l'éloigner.

14. Dispositif ophtalmologique (1) selon l'une quelconque des revendications 1 à 13, **caractérisé en ce que** le dispositif de déplacement (15) et le projecteur de lumière (13) sont disposés et accouplés de telle sorte que le projecteur de lumière (13) puisse être déplacé de manière équidistante par rapport à une surface de déplacement plane.

15. Dispositif ophtalmologique (1) selon l'une quelconque des revendications 1 à 14, **caractérisé en ce que** la source de lumière (12) comprend un laser femtosecondes.

16. Dispositif ophtalmologique (1) selon la revendication 15, **caractérisé en ce que** le laser femtosecondes est réalisé sous forme de laser à fibre.

17. Dispositif ophtalmologique (1) selon l'une quelconque des revendications 1 à 16, **caractérisé en ce que** la source de lumière (12) est connectée au projecteur de lumière (13) par le biais d'une conduite de transport optique (123) qui est réalisée sous la forme d'un cristal photonique.

18. Dispositif ophtalmologique (1) selon l'une quelconque des revendications 1 à 16, **caractérisé en ce que** la source de lumière comprend un oscillateur optique (121) et **en ce que** l'oscillateur optique (121) est connecté par le biais d'un expanseur (122), par le biais d'une conduite de transport optique (123) et par le biais d'un compresseur optique (124) au projecteur de lumière (13), la conduite de transport optique (123) comprenant un amplificateur optique.

19. Dispositif ophtalmologique (1) selon la revendication 18, **caractérisé en ce que** la conduite de transport optique (123) est réalisée sous la forme d'un cristal photonique.

20. Dispositif ophtalmologique (1) selon l'une quelconque des revendications 1 à 19, **caractérisé en ce que** les moyens de fixation (11) sont disposés de manière à fixer le dispositif ophtalmologique (1) sur l'oeil (2) par une dépression.

21. Dispositif ophtalmologique (1) selon l'une quelconque des revendications 1 à 20, **caractérisé en ce qu'**il comprend des moyens de support (7) pour fixer le dispositif ophtalmologique (1) de manière mobile sur un objet externe (8) par rapport au dispositif ophtalmologique.

22. Dispositif ophtalmologique (1) selon l'une quelconque des revendications 1 à 21, **caractérisé en ce qu'**il est disposé de manière à déplacer le foyer (F) de telle sorte qu'un lambeau de tissu (23) qui reste connecté à l'oeil (2) dans une région résiduelle (26), soit séparé du tissu restant de l'oeil (21) par l'ablation tissulaire à l'intérieur du tissu oculaire (21).
